# EUROPEAN PATENT SPECIFICATION

(11) **EP 1 982 983 B1**
(45) Date of publication and mention of the grant of the patent: **17.08.2011**
(21) Application number: 08012788.9
(22) Date of filing: 26.06.2006
(51) Int. Cl.: C07D 403/12

(54) **Improved process for producing moxonidine**
Verbessertes Verfahren zur Herstellung von Moxonidin
Processus amélioré pour la production de moxonidine

(43) Date of publication of application: 22.10.2008
(62) Divisional of application: 06013133.1
(73) Proprietor: CHEMAGIS LTD., 51200 Bnei-Brak (IL)
(72) Inventor: Naddaka, Vladimir, 71338 Lod (IL); Klopfer, Eyal, 64955 Tel Aviv (IL); Saeed, Shady, 33266 Haifa (IL); Davidi, Guy, 40500 Even-Yehuda (IL); Ostrovsky, Elena, 75233 Rishon-LeZion (IL); Arad, Oded, 76303 Rechovot (IL); Kaspi, Joseph, 53201 Givatayim (IL)
(74) Representative: Albrecht, Thomas

(56) References cited:
- WO-A-2004/037795
- DE-A1- 2 849 537
- DE-A1- 2 937 023
- US-A- 4 323 570
- MINEO SANEYOSHI, SHIN'ICHI WATANABE: CHEMICAL AND PHARMACEUTICAL BULLETIN, vol. 36, no. 7, 1988, pages 2673-2673, XP001249009

## Description

### FIELD OF THE INVENTION

The present invention relates to the field of organic chemistry and more particularly to improved process for producing Moxonidine.

### BACKGROUND OF THE INVENTION

Moxonidine (4-chloro-5-(imidazoline-2-ylamino)-6-methoxy-2-methylpyrimidine), has the structural formula (I) below and is used as an antihypertensive drug.

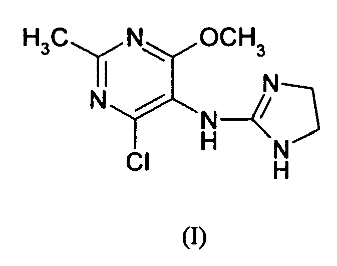

Moxonidine was approved for use in Germany in 1991 and is currently commercially available in Europe, e.g., in Germany, Austria and the UK.

U.S. patent No. 4,323,570 (hereinafter the '570 patent) describes a method of preparing Moxonidine (I) by reacting 4,6-dichloro-2-methyl-5-(1-acetyl-2-imidazolin-2-yl)-aminopyrimidine (hereinafter DMAIA) of formula (II) with about 2 equivalents of sodium methoxide in methanol under reflux, as depicted in Scheme 1.

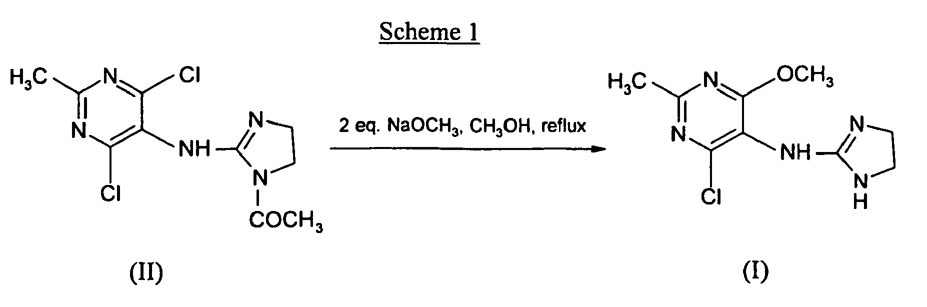

According to example 3 of the '570 patent, Moxonidine is obtained by crystallization from nitromethane.

Czech patent No. 294649 (hereinafter the '649 patent) describes a method of preparing Moxonidine from DMAIA by methanolysis reaction using alkali metal carbonates e.g., potassium carbonate at 47-52°C or sodium bicarbonate at 65°C.

German patent application DE 29 37 023 A1 describes substituted 5-(2-imidazolin-2-yl)-aminopyrimidines of the general formula (I) 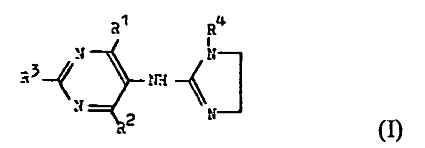 wherein R¹, R² and R³, which may be the same or different, represent a hydrogen or halogen atom, an alkoxy, alkylthio or alkyl group having 1 to 4 carbon atoms or a cycloalkyl group having 3 to 5 carbon atoms, wherein at least one of the substituents R¹, R² and R³ represents an alkylthio or a cycloalkyl group, and R⁴ represents a hydrogen atom or an aliphatic or aromatic acyl group, as well as their physiologically acceptable acid addition salts.

German patent application DE 28 49 537 A1 describes substituted 5-(2-imidazolin-2-yl)-aminopyrimidines of the general formula (I) 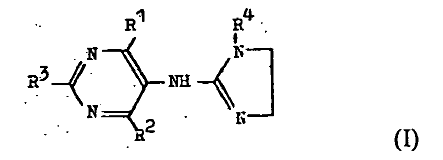 wherein R¹, R² and R³, which may be the same or different, represent a hydrogen or halogen atom, an alkoxy or alkyl group having 1 to 4 carbon atoms and R⁴ represents a hydrogen atom or an aliphatic or aromatic acyl group as well as their physiologically acceptable acid addition salts.

International patent application WO 2004/037795 A1 describes a method for producing 2-amino-4-chloro-6-alkoxypyrimidines by reacting 2-amino-4,6-dichloropyrimidine with an alkali alcoholate or a mixture of alkali hydroxides and an alcohol in a polar aprotic solvent (mixture), whereupon the solvent is distilled off to > 30 percent and the product is precipitated by adding water during or following the distillation process. According to this method, in which especially acetone is used as a polar aprotic solvent and which can be carried out at temperatures between 5 and 60 °C, allows 2-amino-4-chloro-6-alkoxypyrimidines and, in particular, 2-amino-4-chloro-6-methoxypyrimidine to be produced in an economical and environmentally friendly manner while obtaining high yields and purity.

Mineo Saneyoshi et al. (Chemical and Pharmaceutical Bulletin, Vol. 36, No. 7, 1988, p. 2673, XP001249009) describe synthetic nucleosides and nucleotides as well as the synthesis of 5-alkylcytidines from 5-alkylbarbituric acids.

Boiling sodium methoxide is highly corrosive and toxic and therefore there is a need in the art for an improved process for preparing Moxonidine that will use less toxic and corrosive reagents, e.g., sodium hydroxide and potassium hydroxide at a considerably lower concentration (e.g., 1 mole equivalent instead of 2 mole equivalents), and using lower reaction temperatures.

### SUMMARY OF THE INVENTION

The inventors of the present invention have discovered that it is possible to use sodium methoxide for converting 4,6-dichloro-2-methyl-5-(1-acetyl-2-imidazolin-2-yl)-aminopyrimidine (DMAIA) into Moxonidine in much milder conditions (ambient temperature and relatively low molar excess of the base) that those used according to the prior art.

The present invention provides an improved process for producing Moxonidine of formula (I) in high purity and yield, the process comprising:
reacting DMAIA with sodium methoxide in an organic solvent at ambient temperature;
quenching the reaction mixture with water;
isolating Moxonidine; and
optionally purifying Moxonidine by crystallization
wherein the organic solvent
is selected from the group consisting of methanol,
dimethyl sulfoxide (DMSO), and a mixture thereof.

Disclosed is also a more convenient and environmentally friendly process for purifying Moxonidine by crystallization, wherein the solvent used is other than nitromethane, the process comprising:
mixing the crude Moxonidine with an organic solvent;
heating to an elevated temperature;
allowing the mixture to cool sufficiently to enable crystallization; and
isolating the crystals, washing and drying.

According to another embodiment of the present invention, the crude Moxonidine is obtained by the process described herein in at least 88% overall yield, preferably in a yield higher than 90.5%. The crystallized Moxonidine is obtained having a purity of at least 98%, preferably having a purity equal to or greater than 99.5%, and more preferably having a purity equal to or greater than 99.8%.

### DESCRIPTION OF THE PREFERRED EMBODIMENTS

While searching for an improved process for preparing Moxonidine, the inventors of the present invention have reproduced example 3 of the '570 patent and example 1 of the '649 patent and found that in both cases the crude Moxonidine contained substantial levels of the impurities 4,6-dimethoxy-2-methyl-5-(2-imidazolin-2-yl)-aminopyrimidine of formula (III) and 4,6-dichloro-2-methyl-5-(2-imidazolin-2-yl)-aminopyrimidine of formula (IV), as described in reference examples 1 and 2 respectively.

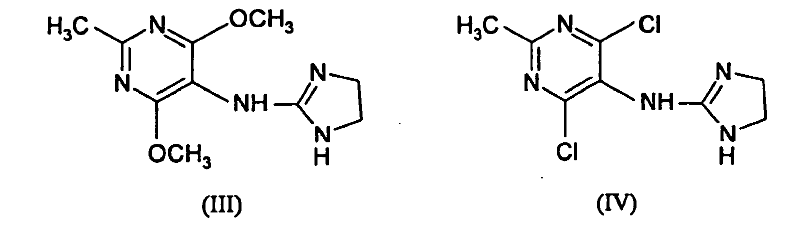

It is apparent to those skilled in the art that purifying crude Moxonidine from these impurities, namely compounds (III) and (IV), may not be an easy task to achieve, and the purified Moxonidine is liable to be obtained in a relatively low yield. Therefore, there is a need in the art for an improved process for producing Moxonidine in high purity and yield, wherein the content of the impurities is minimal.

According to one embodiment of the present invention, methanol is used as a solvent and the volume of methanol is 4-12 ml relative to 1 g of DMAIA, preferably the volume of methanol is 8 ml relative to 1 g of DMAIA.

The present invention provides an improved process for producing Moxonidine, the process comprising:
reacting DMAIA with sodium methoxide at ambient temperature in an organic solvent;
quenching the reaction mixture with water;
isolating Moxonidine; and
optionally purifying Moxonidine by crystallization,
wherein the organic solvent used in the reaction is selected from the group consisting of methanol, dimethyl sulfoxide (DMSO), and mixtures thereof.

According to yet another embodiment of the present invention, the volume of dimethyl sulfoxide (DMSO) is about 10 ml relative to 1 g of DMAIA.

According to yet another embodiment of the present invention, sodium methoxide is used in the reaction and the amount of sodium methoxide is about 0.9-1.7 equivalents relative to one mole of DMAIA, preferably 1.1 equivalents.

According to yet another embodiment of the present invention, the reaction may be stopped after most of the unwanted intermediate 4,6-dichloro-2-methyl-5-(2-imidazolin-2-yl)-aminopyrimidine of formula (IV) has disappeared, as monitored by HPLC.

According to yet another embodiment of the present invention, after reaction completion, water is added to the reaction mixture and crude Moxonidine is isolated from the resulting suspension by filtration. The crude Moxonidine is then washed with water and with 2-propanol and dried.

Also disclosed is a process for purifying Moxonidine by crystallization, wherein the solvent used is other than nitromethane, the process comprising:
mixing the crude Moxonidine with a solvent;
heating to an elevated temperature;
allowing the mixture to cool sufficiently to enable crystallization; and
isolating the crystals, washing and drying.

Typically, the crystallization solvent is a class 3 solvent, e.g., acetone, 1-propanol, 2-propanol, 1-butanol, 2-butanol, dimethyl sulfoxide (DMSO), and a mixture thereof.

According to yet another embodiment of the present invention, the crude Moxonidine is obtained by the process described herein in at least 88% overall yield, preferably in a yield higher than 90.5%. The crystallized Moxonidine is obtained having a purity of at least 98%, preferably having a purity equal to or greater than 99.5%, and more preferably having a purity equal to or greater than 99.8%.

### Examples

The following Examples 1-3 and 9 illustrate the process of the present invention; Examples 10-32 are provided for reference and/or comparison only.

HPLC measurements of Moxonidine samples were performed using HPLC system, equipped with Phenomenex Luna 5µ C8(2) (4.6 x 250 mm) column, and a UV detector operated on 230 nm. Analyses were performed using the following mobile phase, at flow rate of 1.2 ml/minute. Eluent A: 10 mM pentanesulfonic acid, pH = 3.0 with H₂SO₄. Eluent B: acetonitrile. Gradient (A/B, v/v): 0 min (94/6), 4 min (94/6), 20 min (64/36), 50 min (64/36).

### Reference Example 1 - Preparation of Moxonidine by reaction of 4,6-dichloro-2-methyl-5-(1-acetyl-2-imidazolin-2-yl)-aminopyrimidine (DMAIA) with 2.02 equivalents of sodium methoxide at boiling temperature

4,6-dichloro-2-methyl-5-(1-acetyl-2-imidazolin-2-yl)-aminopyrimidine (10 g, 0.0347 mol) was mixed with a solution of sodium methoxide (3.78 g, 0.07 mol, 2.02 equiv.) in 35 ml of methanol and boiled for 2 hours. Then, water (100 ml) was added and the reaction mixture was cooled to ambient temperature. A colorless product was collected by filtration, washed with water and dried at 50° C overnight to yield crude Moxonidine containing 6.5% of impurity (III) and 0.61 % of impurity (IV).

### Reference Example 2 - Preparation of Moxonidine by reaction of DMAIA with 2 molar equivalents of potassium carbonate at 47-52° C.

Potassium carbonate (9.6 g, 0.0694 mol, 2 molar equiv.) was added to a suspension of DMAIA (10 g, 0.0347 mol) in methanol (80 ml) and the mixture was heated at 47-52° C for 5 hours. Then, the reaction mixture was cooled to ambient temperature and acetic acid (10 ml) and water (70 ml) were added. After stirring for half an hour, 25% solution of ammonium hydroxide (10 ml) was added and the mixture was stirred for one hour. A colorless precipitate was collected by filtration, washed with water and dried at 50° C overnight to yield crude Moxonidine containing 0.07% of impurity (III) and 0.40% of impurity (IV).

### Example 1 - Preparation of Moxonidine by reaction of DMAIA with 1.1 equivalents of sodium methoxide at ambient temperature

Sodium methoxide (25% solution in methanol, 7.7 ml, 0.0382 mol, 1.1 equiv.) was added at ambient temperature to a suspension of DMAIA (10 g, 0.0347 mol) in methanol (50 ml) and the reaction mixture was stirred at ambient temperature for 24 hours. Then, water (100 ml) was added and the reaction mixture was cooled to ambient temperature. A colorless product was collected by filtration, washed with water (3 x10 ml) and 2-propanol (3 x10 ml) and dried at 50° C overnight to give 7.4 g of crude Moxonidine in 88.3% yield, having a purity of 99.17% (by HPLC), containing 0.04% of impurity (III) and 0.71% of impurity (IV).

### Examples 2 - 9

The same reaction, which is provided in example 1, was carried out using sodium methoxide in methanol at different reaction conditions, or alternatively using other solvents than methanol, as depicted in Table 1

**Table 1 - Preparation of crude Moxonidine by reaction of DMAIA with sodium methoxide in methanol and other solvents**

| Ex No | DMAIA, g (mol) | Solvent, (ml) | Base, (mol, equiv.) | Temp., °C | Time, *h* | Crude yield g (%) | Purity, % | % of (III) | % of (IV) |
|---|---|---|---|---|---|---|---|---|---|
| 2 | 10(0.035) | MeOH (50) | CH₃ONa (0.035, 1.0) | 25 | 24 | 7.4 (88.3) | 98.27 | 0.02 | 1.56 |
| 3 | 10(0.035) | MeOH (100) | CH₃ONa (0.059,1.7) | 25 | 18 | 7.25 (86.5) | 99.5 | 0.06 | 0.44 |
| 4 | 10(0.035) | MeOH (35) | CH₃ONa (0.035, 1.0) | 65 | 1.5 | 7.4 (88.3) | 96.74 | 0.05 | 3.21 |
| 5 | 5 (0.017) | MeOH (50) | CH₃ONa (0.016,0.9) | 0 | 48 | 3.25 (77.6) | 98.55 | - | 1.45 |
| 6 | 5 (0.017) | MeOH (30) | CH₃ONa (0.017, 1.0) | 0 | 48 | 3.55 (84.7) | 99.35 | 0.09 | 0.48 |
| 7 | 5 (0.017) | THF (50) | CH₃ONa (0.019, 1.1) | 25 | 30 | 3.2 (76.4) | 92.48 | 0.10 | 7.44 |
| 8 | 5 (0.017) | Toluene (50) | CH₃ONa (0.019, 1.1) | 25 | 30 | 2.8 (66.8) | 94.76 | 0.20 | 4.86 |
| 9 | 5 (0.017) | DMSO (50) | CH₃ONa (0.019, 1.1) | 25 | 30 | 3.2 (76.4) | 98.23 | 0.44 | - |

### Example 10 - Preparation of Moxonidine by reaction of DMAIA with 1.0 molar equivalent of potassium carbonate

A mixture of DMAIA (5 g, 0.0174 mol) and potassium carbonate (2.4 g, 0.0174 mol, 1.0 molar equiv.) in methanol (40 ml) was heated at 45-50° C for 18 hours. Then, the reaction mixture was cooled to ambient temperature and acetic acid (4 ml) and water (35 ml) were added. After stirring for half an hour, 25% solution of ammonium hydroxide (4 ml) was added and the mixture was stirred for one hour. A colorless precipitate was collected by filtration, washed with water and dried at 50°C overnight to give 3.8 g of crude Moxonidine in 90.7% yield, having a purity of 99.23% (by HPLC), containing 0.10% of impurity (III) and 0.67% of impurity (IV).

### Examples 11-12

The same reaction, which is provided in example 10, was carried out using potassium carbonate in methanol at different reaction conditions, as depicted in Table 2.

**Table 2 - Preparation of crude Moxonidine by reaction of DMAIA with potassium carbonate**

| Ex No | DMAIA, g (mol) | Solvent, (ml) | Base, (mol, equiv.) | Temp., °C | Time, *h* | Crude yield g (%) | Purity, % | % of (III) | % of (IV) |
|---|---|---|---|---|---|---|---|---|---|
| 11 | 5 (0.017) | MeOH (40) | K₂CO₃ (0.017, 1.0) | 65 | 3 | 3.8 (90.7) | 99.0 | 1.0 | - |
| 12 | 5 (0.017) | MeOH (40) | K₂CO₃ (0.01,0.6) | 65 | 4 | 3.4 (81.1) | 99.33 | 0.6 | 0.07 |

### Example 13 - Preparation of Moxonidine by reaction of DMAIA with 1.5 equivalents of 47% aqueous solution of sodium hydroxide

A mixture of DMAIA (5 g, 0.0174 mol) and 47% aqueous solution of sodium hydroxide (1.25 g, 0.026 mol, 1.5 equiv.) in methanol (40 ml) was stirred at 40-50°C for 10 hours. Then, the reaction mixture was cooled to ambient temperature and water (50 ml) was added. After stirring for one hour, a colorless precipitate was collected by filtration, washed with water (3 x10 ml) and 2-propanol (3 x10 ml) and dried at 50°C overnight to yield 3.7 g of crude Moxonidine in 88.3% yield, having a purity of 99.52% (by HPLC), containing 0.04% of impurity (III) and 0.44% of impurity (IV).

### Examples 14 -16

The same reaction, which is provided in example 13, was carried out using sodium hydroxide solution in methanol at different reaction conditions, as depicted in Table 3.

**Table 3 - Preparation of crude Moxonidine by reaction of DMAIA with sodium hydroxide solution**

| Ex No | DMAIA, g (mol) | Solvent, (ml) | Base, (mol, equiv.) | Temp., °C | Time, *h* | Crude yield g (%) | Purity, % | % of (III) | % of (IV) |
|---|---|---|---|---|---|---|---|---|---|
| 14 | 5 (0.017) | MeOH (40) | 47% aq. NaOH sol. (0.019, 1.1) | 25 | 24 | 3.7 (88.3) | 98.54 | - | 1.46 |
| 15 | 5 (0.017) | MeOH (40) | 47% aq. NaOH sol. (0.023, 1.3) | 55-60 | 5 | 3.7 (88.3) | 99.47 | 0.08 | 0.45 |
| 16 | 5 (0.017) | MeOH (40) | 47% aq. NaOH sol. (0.019, 1.1) | 65 | 2 | 3.4 (81.1) | 98.86 | 0.13 | 1.01 |

### Example 17 - Preparation of Moxonidine by reaction of DMAIA with 1.3 equivalents of 85% potassium hydroxide powder at ambient temperature

A mixture of DMAIA (5 g, 0.0174 mol) and 85% potassium hydroxide powder (1.5 g, 0.0226 mol, 1.3 equiv.) in methanol (40 ml) was stirred at ambient temperature for 24 hours. Then, water (50 ml) was added and the mixture was stirred for one hour. A colorless precipitate was collected by filtration, washed with water (3 x10 ml) and 2-propanol (3 x10 ml) and dried at 50°C overnight to give 3.7 g of crude Moxonidine in 88.3% yield, having a purity of 99.49% (by HPLC), containing 0.01% of impurity (III) and 0.50% of impurity (IV).

### Examples 18 -19

The same reaction, which is provided in example 17, was carried out using 85% potassium hydroxide powder in methanol at different reaction conditions, as depicted in Table 4.

**Table 4 - Preparation of crude Moxonidine by reaction of DMAIA with solid potassium hydroxide**

| No | DMAIA, g (mol) | Solvent, (ml) | Base, (mol, equiv.) | Temp., °C | Time, *h* | Crude yield g (%) | Purity, % | % of (III) | % of (IV) |
|---|---|---|---|---|---|---|---|---|---|
| 18 | 5 (0.017) | MeOH (40) | 85% KOH powder (0.019, 1.1) | 25 | 30 | 3.5 (83.5) | 98.21 | 0.02 | 1.77 |
| 19 | 5 (0.017) | MeOH (40) | 85% KOH powder (0.026, 1.5) | 25 | 12 | 3.7 (88.3) | 99.50 | - | 0.50 |

### Example 20 - Preparation of Moxonidine by reaction of DMAIA with 45% aqueous potassium hydroxide solution at ambient temperature

A mixture of DMAIA (5 g, 0.0174 mol) and 45% aqueous potassium hydroxide solution (1.8 g, 0.029 mol, 1.7 equiv.) in methanol (40 ml) was stirred at ambient temperature for 10 hours. Then, water (50 ml) was added and the mixture was stirred for one hour. A colorless precipitate was collected by filtration, washed with water (3 x10 ml) and 2-propanol (3 x10 ml) and dried at 50° C overnight to give 3.7 g of crude Moxonidine in 88.3% yield, having a purity of 99.45% (by HPIC), containing 0.03% of impurity (III) and 0.52% of impurity (IV).

### Examples 21 -25

The same reaction, which is provided in example 20, was carried out using aqueous potassium hydroxide in methanol at different reaction conditions, as deploted in Table 5.

**Table 5 - Preparation of crude Moxonidine by reaction of DMAIA with aqueous potassium hydroxide**

| Ex. No | DMAIA, g (mol) | Solvent, (ml) | Base, (mol, equiv.) | Temp., °C | Time, *h* | Crude Yield g (%) | Purity, % | % of (III) | % of (IV) |
|---|---|---|---|---|---|---|---|---|---|
| 21 | 5 (0.017) | MeOH (40) | 45% aq. KOH sol. (0.023, 1.3) | 65 | 3 | 3.6 (85.9) | 99.34 | 0.19 | 0.47 |
| 22 | 5 (0.017) | MeOH (40) | 45% aq. KOH sol. (0.019, 1.1) | 65 | 2 | 3.4 (81.1) | 98.28 | 0.20 | 1.52 |
| 23 | 5 (0.017) | MeOH (40) | 45% aq. KOH sol. (0.023, 1.3) | 25 | 11 | 3.6 (85.9) | 98.71 | 0.01 | 1.28 |
| 24 | 5 (0.017) | MeOH (40) | 45% aq. KOH sol. (0.026, 1.5) | 25 | 10 | 3.6 (85.9) | 99.04 | 0.02 | 0.93 |
| 25 | 5 (0.017) | MeOH (40) | 45% aq. KOH sol. (0.029, 1.7) | 25 | 10 | 3.7 (88.3) | 99.17 | 0.02 | 0.81 |

### Examples 26 -29

The same reaction, which is provided in example 17, was carried out using sodium bicarbonate powder in methanol, optionally with addition of small volume of water (example 29), at different reaction conditions, as depicted in Table 6.

**Table 6 - Preparation of crude Moxonidine by reaction of DMAIA with sodium bicarbonate**

| Ex. No | DMAIA, g (mol) | Solvent, (ml) | Base, (mol, equiv.) | Temp., °C | Time, *h* | Crude Yield g (%) | Purity, % | % of (III) | % of (IV) |
|---|---|---|---|---|---|---|---|---|---|
| 26 | 5 (0.017) | MeOH (40) | NaHCO₃ (0.017, 1.0) | 65 | 14.5 | 3.6 (85.9) | 98.71 | 0.56 | 0.65 |
| 27 | 5 (0.017) | MeOH (40) | NaHCO₃ (0.023, 1.3) | 65 | 17.5 | 3.7 (88.3) | 97.82 | 1.32 | 0.86 |
| 28 | 5 (0.017) | MeOH (40) | NaHCO₃ (0.026, 1.5) | 40-50 | 24 | 2.9 (71.1) | 97.85 | 1.87 | 0.14 |
| 29 | 5 (0.017) | MeOH (40), water (2) | NaHCO₃ (0.029, 1.7) | 65 | 6 | 3.7 (88.3) | 98.59 | 0.28 | 1.13 |

### Examples 30 -31

The same reaction, which is provided in example 17, was carried out using potassium bicarbonate powder in methanol, optionally with addition of small volume of water (example 31), at different reaction conditions, as depicted in Table 7.

**Table 7 - Preparation of crude Moxonidine by reaction of DMAIA with potassium bicarbonate**

| Ex. No | DMAIA, g (mol) | Solvent, (ml) | Base, (mol, equiv.) | Temp., °C | Time, *h* | Crude Yield g (%) | Purity, % | % of (III) | % of (IV) |
|---|---|---|---|---|---|---|---|---|---|
| 30 | 5 (0.017) | MeOH (40) | KHCO₃ (0.026, 1.5) | 65 | 9 | 3.6 (85.9) | 99.39 | 0.53 | 0.08 |
| 31 | 5 (0.017) | MeOH (40), water (2) | KHCO₃ (0.026, 1.5) | 65 | 10 | 3.7 (88.3) | 99.20 | 0.35 | 0.45 |

### Example 32 - Preparation of Moxonidine by reaction of DMAIA with a mixture of aqueous potassium hydroxide and solid potassium carbonate at ambient temperature

A mixture of DMAIA (5 g, 0.0174 mol) and 45% potassium hydroxide solution (0.023 mol, 1.3 equiv.) and solid potassium carbonate (0.004 mol, 0.2 equiv.) in methanol (40 ml) was stirred at ambient temperature for 16 hours. Then, water (50 ml) was added and the mixture was stirred for one hour. A colorless precipitate was collected by filtration, washed with water (3 x10 ml) and 2-propanol (3 x10 ml) and dried at 50° C overnight to yield 3.6 g of crude Moxonidine in 85.9% yield, having a purity of 99.07% (by HPLC), containing 0.03% of impurity (III) and 0.90% of impurity (IV).

### Example 33 - Crystallization of Moxonidine from DMSO

Crude Moxonidine (2.0 g), having a purity of 99.12% and containing 0.03% of impurity (III) and 0.85% of impurity (IV), was mixed with DMSO (10 ml) and heated to a temperature of about 90°C to obtain a clear solution. The solution was cooled to ambient temperature and the thus formed crystals were obtained by filtration and washed with cold 2-propanol and dried under reduced pressure. 1.7 g of crystallized Moxonidine were obtained in 85% yield having a purity by HPLC of 99.91%, and containing 0.02% of impurity (III) and 0.06% of impurity (IV)

Table 8 contains the results of crystallization from different solvents.

**Table 8. Crystallization of crude Moxonidine from different solvents**

| Ex. No. | Solvent | volume, ml | Moxonidine crude, g | Yield, % | Purity, % | % of (III) | % of (IV) |
|---|---|---|---|---|---|---|---|
| 34 | 1-propanol | 48 | 2 | 85 | 99.61 | 0.04 | 0.30 |
| 35 | 2-propanol | 80 | 2 | 80 | 99.35 | 0.03 | 0.62 |
| 36 | 1-butanol | 52 | 2 | 85 | 99.70 | 0.05 | 0.25 |

## Claims

1. A process for preparing Moxonidine, comprising:
reacting 4,6-dichloro-2-methyl-5-(1-acetyl-2-imidazolin-2-yl)amino-pyrimidine (DMAIA) with sodium methoxide at ambient temperature in an organic solvent selected from methanol, dimethyl sulfoxide (DMSO) and mixtures thereof;
quenching the reaction mixture with water;
isolating Moxonidine; and
optionally recrystallizing Moxonidine from an organic solvent.

2. The process of claim 1, wherein between 0.9-1.7 equivalents of sodium methoxide are used with respect to 1 equivalent of DMAIA.

3. The process of claim 1, wherein water is added to the reaction mixture after reaction completion, and the crude Moxonidine is isolated from the resulting suspension by filtration, washed with water and 2-propanol and dried.

4. The process of claim 1, wherein crude non-crystallized Moxonidine is obtained in at least 88% yield.

5. The process of claim 4, wherein crude non-crystallized Moxonidine is obtained in a yield higher than 90.5%.

## Patentansprüche

1. Verfahren zur Herstellung von Moxonidin, umfassend:
Umsetzen von 4,6-Dichlor-2-methyl-5-(1-acetyl-2-imidazolin-2-yl)amino-pyrimidin (DMAIA) mit Natriummethoxid bei Umgebungstemperatur in einem organischen Lösungsmittel, ausgewählt aus Methanol, Dimethylsulfoxid (DMSO) und Gemischen davon;
Abschrecken des Reaktionsgemisches mit Wasser;
Isolieren von Moxonidin und
gegebenenfalls Umkristallisieren von Moxonidin aus einem organischen Lösungsmittel.

2. Verfahren gemäß Anspruch 1, wobei zwischen 0,9-1,7 Äquivalente Natriummethoxid bezüglich 1 Äquivalent DMAIA verwendet werden.

3. Verfahren gemäß Anspruch 1, wobei Wasser dem Reaktionsgemisch nach Reaktionsbeendigung zugesetzt wird und das rohe Moxonidin aus der resultierenden Suspension durch Filtration isoliert wird, mit Wasser und 2-Propanol gewaschen wird und getrocknet wird.

4. Verfahren gemäß Anspruch 1, wobei rohes nichtkristallisiertes Moxonidin in wenigstens 88% Ausbeute erhalten wird.

5. Verfahren gemäß Anspruch 4, wobei rohes nichtkristallisiertes Moxonidin in einer Ausbeute von höher als 90,5% erhalten wird.

## Revendications

1. Procédé de préparation de la Moxonidine, comprenant:
la réaction de 4,6-dichloro-2-méthyl-5-(1-acétyl-2-imidazolin-2-yl)amino-pyridine (DMAIA) avec du méthoxyde de sodium à la température ambiante dans un solvant organique choisi parmi le méthanol, le diméthylsulfoxyde (DMSO) et leurs mélanges;
le trempage du mélange réactionnel avec de l'eau;
l'isolement de la Moxonidine;
éventuellement la recristallisation de la Moxonidine dans un solvant organique.

2. Procédé selon la revendication 1, dans lequel entre environ 0,9 et 1,7 équivalents de méthoxyde de sodium sont utilisés par rapport à 1 équivalent de DMAIA.

3. Procédé selon la revendication 1, dans lequel de l'eau est ajoutée au mélange au terme de la réaction, et la Moxonidine brute est isolée de la suspension résultante par filtration, lavée avec de l'eau est séchée.

4. Procédé selon la revendication 1, dans lequel la Moxonidine brute non-cristallisée est obtenue avec un rendement d'au moins 88%.

5. Procédé selon la revendication 4, dans lequel la Moxonidine brute non-cristallisée est obtenue avec un rendement supérieur à 90,5%.
